# EUROPÄISCHE PATENTSCHRIFT

(11) **EP 0 779 310 B1**
(45) Veröffentlichungstag und Bekanntmachung des Hinweises auf die Patenterteilung: **06.05.1999**
(21) Anmeldenummer: 97103009.3
(22) Anmeldetag: 17.07.1993
(51) Int. Cl.: C08G 18/08, C08G 18/42, A61K 7/06, A61K 9/20, A61K 9/32, C09J 175/06

(54) **Verwendung von wasserlöslichen oder in Wasser dispergierbaren Polyurethanen als Hilfsmittel in kosmetischen und pharmazeutischen Zubereitungen und Polyurethane, die Polymilchsäurepolyole einpolymerisiert enthalten**
Use of water-soluble or water-dispersible polyurethanes as auxiliary agents in cosmetic and pharmaceutical preparations and polyurethanes containing polylactic acid polyols incorporated by polymerization
Utilisation de polyuréthanes solubles dans l'eau ou dispersibles dans l'eau comme adjuvants dans des préparations cosmétiques et pharmaceutiques, et polyuréthanes renfermant des polyolpolylactiques incorporés par polymérisation

(30) Priorität: 29.07.1992 DE 4225045
(43) Veröffentlichungstag der Anmeldung: 18.06.1997
(62) Teilanmeldung aus: 93915942.2
(73) Patentinhaber: BASF AKTIENGESELLSCHAFT, 67056 Ludwigshafen (DE)
(72) Erfinder: Kim, Son Nguyen, Dr., 69502 Hemsbach (DE); Sanner, Axel, Dr., 67227 Frankenthal (DE); Sperling-Vietmeier, Karin, Dr., 67433 Neustadt (DE)

(56) Entgegenhaltungen:
- EP-A- 0 039 162
- EP-A- 0 295 055
- US-A- 3 835 081
- US-A- 3 975 350

## Beschreibung

Die Erfindung betrifft die Verwendung von wasserlöslichen oder in Wasser dispergierbaren Polyurethanen aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat
mit einer bevorzugten Glastemperatur von mindestens 15°C und Säurezahlen von 12 bis 150 oder den Salzen dieser Polyurethane als Hilfsmittel in kosmetischen und pharmazeutischen Zubereitungen und wasserlösliche oder in Wasser dispergierbare Polyurethane.

Polyurethane, die zumindest teilweise biologisch abbaubar sind und Hydroxycarbonsäureeinheiten einpolymerisiert enthalten, sind bereits bekannt. Sie sind entweder wasserunlöslich, wie das Polyurethan aus Polymilchsäurediol und Diisocyanat, das aus der SU-A-1 016 314 bekannt ist, oder sie bilden zu weiche Filme, wie die aus der US-A-4 098 743 und der US-A-4 147 679 bekannten Polyurethane aus Poly(ε-caprolacton-diol)dimethylolpropionsäure und Diisocyanaten.

Wasserlösliche Polyurethane, die Carboxylgruppen aufweisende Diole einpolymerisiert enthalten, sind aus der US-A-3 412 054 und der US-A-3 658 939 bekannt. Sie werden als Klebstoff, Beschichtungsmittel und in Drucktinten verwendet.-Sulfonat- und/oder Carboxylatgruppen enthaltende Polyurethane, die in Wasser dispergierbar sind, sind aus der DE-A-15 70 615 bekannt. Sie werden beispielsweise zur Beschichtung und zum Imprägnieren von Textilien, Leder, Papier, Holz und Metallen verwendet.

In der Kosmetik werden Haarbehandlungsmittel, die beispielsweise als Haarverfestiger oder Haarspray vorliegen, zum Festigen, Strukturverbessern und Formgeben der Haare verwendet. Die Haarbehandlungsmittel bestehen vorwiegend aus einer Lösung von filmbildenden Harzen oder synthetischen Polymeren. Bisher wurden in Haarbehandlungsmitteln hauptsächlich folgende Filmbildner verwendet: Schellack, Homo- und Copolymerisate des N-Vinylpyrrolidons, Copolymerisate von Vinylethern/Maleinsäurehalbestern, von (Meth)acrylsäure oder deren Estern und Amiden und Crotonsäure mit Vinylestern.

Die Haarbehandlungsmittel werden in Form von Lösungen, vorzugsweise als ethanolische Lösungen, durch Sprühen auf die Haare gebracht. Nach dem Verdampfen des Lösemittels werden die Haare an den gegenseitigen Berührungspunkten vom zurückbleibenden Polymer in der gewünschten Form gehalten. Die Polymeren sollen einerseits so hydrophil sein, daß sie aus dem Haar ausgewaschen werden können, andererseits sollen sie hydrophob sein, damit die mit den Polymeren behandelten Haare auch bei hoher Luftfeuchtigkeit ihre Form behalten und nicht miteinander verkleben.

Die bisher bekannten polymeren Filmbildner, wie Polyvinylpyrrolidone zeigen jedoch meistens als Nachteil eine zu hohe Wasseraufnahme bei erhöhter Luftfeuchtigkeit. Diese Eigenschaft führt u.a. zu einem unerwünschten Verkleben der Haare und zu einem Verlust der Festigkeit und damit einem Zusammenbruch der Haarfrisur. Wird andererseits die Widerstandsfähigkeit gegen hohe Luftfeuchtigkeit verbessert, z.B. bei Copolymerisaten aus N-Vinylpyrrolidon und Vinylacetat, so leidet darunter die Elastizität des Films und die Sprödigkeit dieser Filme kann nach der Haarbehandlung sogar zu einem unangenehmen Stauben und einem schuppigen Belag führen. Außerdem wird vor allem die Auswaschbarkeit bei der Reinigung der Haare sehr erschwert. Die obengenannten synthetischen Haarbehandlungsmittel sind aufgrund ihrer hydrolysebeständigen C-C-Kette biologisch nicht abbaubar. Schellack ist dagegen biologisch abbaubar, hat aber viele Nachteile. So sind seine Eigenschaften als Haarbehandlungsmittel im Vergleich zu den Homo- und Copolymerisaten des N-Vinylpyrrolidons schlechter, insbesondere bezüglich der Klebrigkeit, Wasserlöslichkeit und Steifigkeit. Da Schellack ein Naturprodukt ist, sind seine Eigenschaften starken Schwankungen unterlegen.

Der vorliegenden Erfindung liegt die Aufgabe zugrunde, Hilfsmittel für kosmetische und pharmazeutische Zubereitungen zur Verfügung zu stellen.

Diese Aufgabe wird gelöst mit wasserlöslichen oder in Wasser dispergierbaren Polyurethanen aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat
mit Säurezahlen von 12 bis 150 oder den Salzen dieser Polyurethane, wenn sie als Verbindungen der Gruppe a) mindestens 5 mol-% eines Polykondensats aus Milchsäure und einem Polyol der allgemeinen Formel 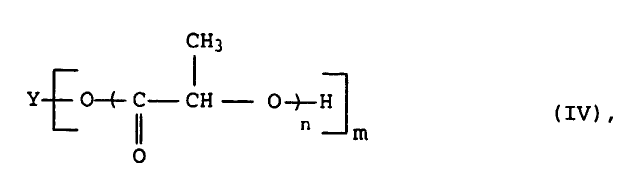 in der
- Y: Rest eines 2- bis 4-wertigen Alkohols,
- n: 1 - 50 und
- m: 1 - 4 bedeutet,
einpolymerisiert enthalten.

Für die erfindungsgemäße Verwendung kommen alle wasserlöslichen oder in Wasser dispergierbaren Polyurethane in Betracht, die die oben angegebenen Komponenten a) bis c) einpolymerisiert enthalten, eine Glastemperatur von bevorzugt mindestens 15°C und Säurezahlen von 12 bis 150 aufweisen sowie die Salze der Polyurethane. Als Verbindungen der Gruppe a) kommen alle die für die Herstellung von Polyurethanen einsetzbaren Verbindungen mit 2 oder mehreren aktiven Wasserstoffatomen pro Molekül in Betracht. Als Verbindungen der Gruppe a) eignen sich beispielsweise Diole, Diamine, Polyesterole, Polyetherole oder Mischungen der genannten Verbindungen, wobei bis zu 3 mol-% der genannten Verbindungen durch Triole oder Triamine ersetzt sein können. Geeignete Diole sind beispielsweise Ethylenglykol, Propylenglykol, Butylenglykol, Neopentylglykol, Polyetherole wie Polyethylenglykole mit Molekulargewichten bis zu 3000, Blockcopolymerisate aus Ethylenoxid und Propylenoxid mit Molekulargewichten nach dem Zahlenmittel von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Vorzugsweise verwendet man aus der Gruppe der Diole und Polyetherole Ethylenglykol, Neopentylglykol, Diethylenglykol, Triethylenglykol, Tetraethylenglykol, Pentaethylenglykol und Hexaethylenglykol.

Geeignete Diamine sind beispielsweise Ethylendiamin, Propylendiamin, 1,4-Diaminobutan und Hexamethylendiamin sowie α,ω-Diamine, die durch Aminierung von Polyalkylenoxiden, insbesondere Polyethylenoxiden mit Ammoniak herstellbar sind.

Als Verbindungen der Gruppe a) kommen außerdem sämtliche Polyesterole in Betracht, die üblicherweise zur Herstellung von Polyurethanen eingesetzt werden, z.B. Umsetzungsprodukte aus Phthalsaure und Diethylenglykol, Isophthalsäure und Butandiol-(1,4), Isophthalsaure/Adipinsäure und Hexandiol-(1,6) sowie aus Adipinsäure und Ethylenglykol.

Insbesondere eignen sich als Polyesterole Poly(α-Hydroxycarbonsäurediole) der Formel 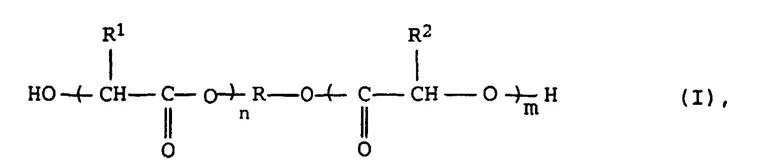 in der
- R¹, R²: H, C₁- bis C₅-Alkyl oder Aryl,
- R: Rest eines zweiwertigen Diols (Alkylenrest) mit 2 bis 8 C-Atomen
- n, m: 1 - 30 bedeuten.

Der Rest R in Formel I bedeutet vorzugsweise -CH₂-CH₂-, 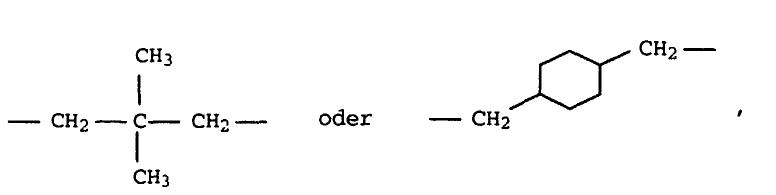 die Reste R¹ und R² stehen vorzugsweise für CH₃.

Geeignete α-Hydroxycarbonsäuren für die Herstellung der Poly-α-Hydroxycarbonsäurediole sind beispielsweise Milchsäure, α-Hydroxybuttersäure, Lactid und Glyoxylsäure. Vorzugsweise setzt man Milchsäure ein, von der sämtliche Isomeren geeignet sind: L,D,DL-Milchsäure.

Zur Herstellung der Polyurethane kann man auch Mischungen von Verbindungen der Gruppe a) einsetzen, z.B. Mischungen aus einem Diol und einem Polyesterol, oder einem Diol und Polyetherolen. In den Mischungen können bis zu 3 mol-% der genannten Verbindungen durch Triole oder Triamine ersetzt sein. Geeignete Triole sind beispielsweise Glycerin, Trimethylolethan oder Trimethylolpropan. Als Triamine eignen sich insbesondere Diethylentriamin oder Dipropylentriamin.

Als Verbindungen der Gruppe b) zur Herstellung der Polyurethane können alle hierfür üblichen Säure- oder Salzgruppen enthaltenden Diole eingesetzt werden. Insbesondere eignen sich Dimethylolpropansäure, Verbindungen der Formel 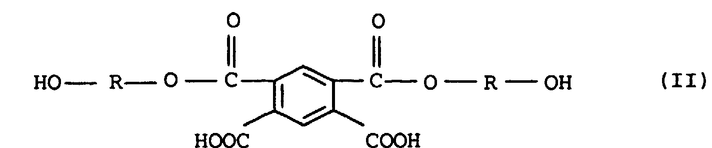 und/oder

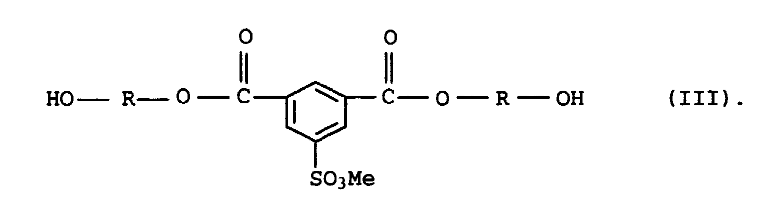

In den Formeln II und III steht R jeweils für eine C₂- bis C₁₈-Alkylengruppe und bedeutet vorzugsweise 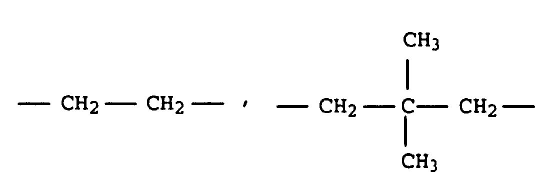 und/oder 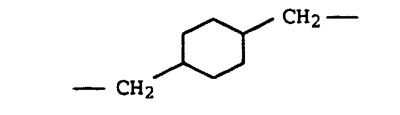 und/oder In Formel III steht Me für Na oder K.

Zur Herstellung der Polyurethane können die üblicherweise verwendeten Di- und Polyisocyanate verwendet werden. Besonders bevorzugt verwendet man als Verbindungen der Gruppe c) Hexamethylendiisocyanat, Isophorondiisocyanat und/oder Toluylendiisocyanat. Wie bei der Herstellung von Polyurethanen üblich, kann man Kettenverlängerer verwenden. Geeignete Kettenverlängerer sind beispielsweise Hexamethylendiamin, Piperazin, 1,2-Diaminocyclohexan, 1,3-Diaminocyclohexan, 1,4-Diaminocyclohexan, Neopentandiamin und 4,4'-Diaminodicyclohexylmethan.

Wasserlösliche oder in Wasser dispergierbare biologisch abbaubare Polyurethane, die als Komponente a) mindestens 5 mol-% eines Polykondensats aus Milchsäure und einem Polyol der allgemeinen Formel 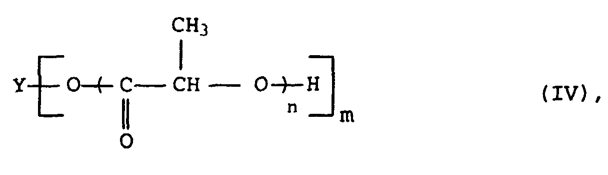 in der
- Y: Rest eines 2- bis 4-wertigen Alkohols,
- n: 1 - 50 und
- m: 1 - 4 bedeutet,
einpolymerisiert enthalten, sind neue Stoffe. Die Verbindungen der Formel IV sind beispielsweise dadurch erhältlich, daß man einen 2- bis 4-wertigen Alkohol mit 1 bis 50 mol Milchsäure verestert. Vorzugsweise verwendet man als Verbindungen der Gruppe a) Umsetzungsprodukte von Diolen mit Milchsäure, wobei man pro Mol Diol bis zu 50 mol, insbesondere 5 bis 30 mol Milchsäure einsetzt. Als Diol eignet sich beispielsweise Ethylenglykol, Propylenglykol, Butylenglykol, Neopentylglykol oder 1,6-Hexandiol sowie Polyetherole, wie Diethylenglykol, Triethylenglykol, Tetraethylenglykol, Pentaethylenglykol, Copolymerisate aus Ethylenoxid und Propylenoxid oder Copolymerisate aus Ethylenoxid, Propylenoxid und Butylenoxid, die die Alkylenoxideinheiten in Form von Blökken oder in statistischer Verteilung angeordnet enthalten können. Die Polyetherole haben Molekulargewichte bis zu 3000, vorzugsweise bis zu 1000. Als Komponente a) zur Herstellung der neuen Polyurethane können die Verbindungen der Formel IV entweder allein verwendet werden oder in Mischung mit anderen, für die Herstellung von Polyurethanen üblicherweise eingesetzten Verbindungen der Komponente a), die oben genannt sind. Sofern Mischungen aus verschiedenen Verbindungen der Gruppe a) in Betracht kommen, setzt man von den Verbindungen der Formel IV mindestens 5, vorzugsweise mindestens 20 mol-% in der Mischung ein.

Die oben bezeichneten neuen Polyurethane sind dadurch erhaltlich, daß man die Verbindungen der Gruppen a) und b) unter einer Inertgasatmosphäre in einem inerten Lösemittel bei Temperaturen von 70 bis 130°C mit den Verbindungen der Gruppe c) umsetzt. Diese Umsetzung kann ggf. in Gegenwart von Kettenverlängerern durchgeführt werden, um Polyurethane mit höheren Molekulargewichten herzustellen. Wie bei der Herstellung von Polyurethanen üblich, werden die Komponenten [(a)+(b)]:(c) im molaren Verhältnis von 0,8 bis 1,1:1 eingesetzt. Die Säurezahl der Polyurethane wird von der Zusammensetzung und der Konzentration der Verbindungen der Komponente (b) in der Mischung aus den Komponenten (a)+(b) bestimmt. Die Polyurethane haben K-Werte nach H. Fikentscher (bestimmt in 0,1 gew.-%igen Lösungen in N-Methylpyrrolidon bei 25°C und pH 7) von 15 - 100, vorzugsweise 25 bis 50.

Ebenfalls neue Stoffe sind wasserlösliche oder in Wasser dispergierbare biologisch abbaubare Polyurethane, die als Komponente b) mindestens 5 mol-% einer Verbindung der Formel III 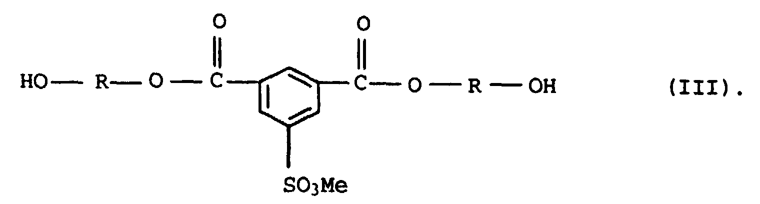 in der R für eine C₂- C₁₈-Alkylengruppe und Me für Na oder K steht, einpolymerisiert enthalten.

Sämtliche oben beschriebenen Polyurethane werden erfindungsgemäß als Hilfsmittel in kosmetischen und pharmazeutischen Zubereitungen verwendet. Für die Anwendung im kosmetischen und pharmazeutischen Bereich werden diejenigen Polyurethane eingesetzt, die Säurezahlen von 12 bis 150, vorzugsweise 30 bis 90 sowie eine Glastemperatur von mindestens 15°C haben. Die Glastemperatur T_{g} kann bis zu 120°C betragen und liegt vorzugsweise in dem Bereich von 30 bis 100°C. Die Glastemperatur T_{g} wird nach ASTM D 3418 bestimmt.

Die Polyurethane sind nach Neutralisation (teilweise oder vollständig) wasserlöslich bzw. ohne Zuhilfenahme von Emulgatoren in Wasser dispergierbar. In aller Regel weisen die Salze der Polyurethane, die durch Neutralisation mit Basen daraus erhältlich sind, eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyl, Calciumoxid, Magnesiumhydroxyd oder Magnesiumcarbonat sowie Ammoniak und Amine verwendet werden. Besonders für den Einsatz in Haarbehandlungsmitteln haben sich zur Neutralisation der Säuregruppen enthaltenden Polyurethane 2-Amino-2-Methylpropanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 20 bis 40 % oder vollständig, d.h. zu 100 % erfolgen.

Sind die erfindungsgemäßen Verbindungen wasserdispergierbar, können sie in Form von wäßrigen Mikrodispersionen mit Teilchendurchmessern von üblicherweise 5 bis 100 nm, insbesondere 10 bis 80 nm, und Feststoffgehalten von üblicherweise 1 bis 40 Gew.-%, insbesondere 3 bis 30 Gew.-%, zur Anwendung gebracht werden. Diese Mikrodispersionen benötigen in der Regel keine Emulgatoren oder Tenside zu ihrer Stabilisierung.

Die von Milchsäurepolyolen abgeleiteten Polyurethane sind zumindest teilweise biologisch abbaubar. Sämtliche Säuregruppen enthaltenden Polyurethane sind am Klärschlamm zu mehr als 90 % eliminierbar (bestimmt nach Zahn-Wellens gemaß DIN 38 412, Teil 25).

Die oben beschriebenen Polyurethane werden außer in der Haarkosmetik auch für Cremes und im Pharmabereich als Tablettenüberzugsmittel und Tablettenbinder verwendet. Die oben beschriebenen neuen Stoffe, die als charakteristische Bestandteile mindestens eine Verbindung der Formel IV einpolymerisiert enthalten, können darüber hinaus noch als Schlichtemittel und als in Wasser löslicher Klebstoff verwendet werden. Für die Verwendung als Klebstoff eignen sich insbesondere solche Polyurethane, die Einheiten der Formel IV einpolymerisiert enthalten und Glastemperaturen unterhalb von 15°C aufweisen. Sofern die oben beschriebenen Polyurethane als Haarbehandlungsmittel verwendet werden, gelangen sie meistens in Form von wäßrigen oder ethanolischen Lösungen zur Anwendung. Der Feststoffgehalt dieser Lösungen beträgt 0,1 bis 30, vorzugsweise 1 bis 15 Gew.-% Polyurethan oder Salz eines Polyurethans.

### Beispiele

### Allgemeine Herstellungsvorschrift

In einem 4-Halskolben, der mit Rührer, Tropftrichter, Thermometer, Rückflußkühler und Vorrichtung für das Arbeiten unter Stickstoff ausgestattet ist, werden die in der Tabelle angegebenen Verbindungen a) und b) im Methylethylketon gelöst. Dazu wird das Reaktionsgemisch auf eine Temperatur von ca. 80°C unter Rühren erhitzt. Sobald sich alles gelöst hat, kühlt man das Reaktionsgemisch auf ca. 50°C ab und tropft unter Rühren das in der Tabelle unter c) jeweils angegebene Diisocyanat zu. Die Reaktionstemperatur steigt dabei an. Bei einer Innentemperatur von 90°C wird das Reaktionsgemisch dann solange gerührt, bis der Isocyanatgruppengehalt des Gemisches praktisch konstant bleibt. Danach kühlt man das Reaktionsgemisch auf eine Temperatur in dem Bereich von 10°C bis 30°C ab und tropft bei dieser Temperatur das in der Tabelle angegebene Diamin langsam zu. Man rührt das Reaktionsgemisch dann noch solange in diesem Temperaturbereich, bis der Isocyanatgruppengehalt auf 0 abgefallen ist. Sofern man keinen Kettenverlängerer zusetzt, werden die restlichen Isocyanatgruppen durch Zusatz von Aminen, z.B. 2-Amino-2-methyl-1-propanol inaktiviert. Man fügt dann Ethanol zu und entfernt den größten Teil des Methylethylketons und des Ethanols unter vermindertem Druck bei ca. 40°C. Das restliche Ethanol wird im Vakuumtrockenschrank bei 50°C entfernt. Man erhält nach dem Trocknen ein elastisches bis sehr hartes Produkt, das in Ethanol sowie in Wasser - vorzugsweise nach der Neutralisation mit einem Amin - löslich bzw. dispergierbar ist.

Anstelle der Zugabe von Ethanol zum Reaktionsgemisch kann man auch Wasser zusetzen und das Reaktionsprodukt neutralisieren, z.B. mit einem Amin. Das als Lösemittel verwendete Methylethylketon kann dann im Vakuum bei 40°C abdestilliert werden, so daß man direkt eine wäßrige Lösung bzw. Dispersion eines säuregruppenenthaltenden Polyurethans mit den in der Tabelle angegebenen Eigenschaften erhält. Die Abkürzungen in der Tabelle haben folgende Bedeutung:
- PEG300:: Polyethylenglykol M_{w} = 300 g/mol
- NPG:: Neopentylglykol
- DMPA:: Dimethylolpropansäure
- IPDI:: Isophorondiisocyanat
- P(IPS/ADS-VI):: Polyesterol mit M_{w} = 1000 g/mol aus Isophthalsäure, Adipinsäure und Hexandiol.
- P(ADS-DEG):: Polyesterol mit M_{w} = 500 g/mol aus Adipinsäure und Diethylenglykol
- P(PS-DEG):: Polyesterol mit M_{w} = 450 g/mol aus Phthalsäure und Diethylenglykol
- P(MIS-EG):: Polymilchsäure-ethylenglykol M_{w} = 500 g/mol.
- P(PMDA-NPG) :: Kondensat aus Pyromellitsäuredianhydrid und Neopentylglykol vom Molekulargewicht M_{w} von ca. 430 mit der Struktur 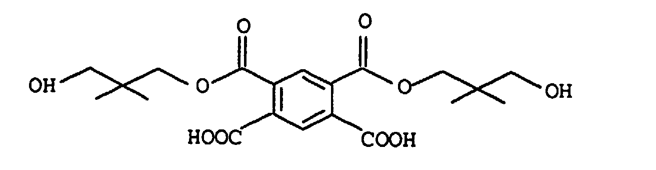
- P(SIPS-NPG):: Kondensat aus 5-Natriumsulfonato-isophthalsäure mit Neopentylglykol vom Molekulargewicht M_{w} ca. 440 und der Struktur 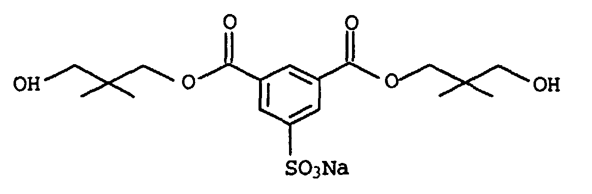
- NMP:: N-Methylpyrrolidon
- EtOH:: Ethanol
- l:: leicht löslich
- disp:: dispergierbar

Die biologische Abbaubarkeit der Polyurethane wurde nach Zahn-Wellens, DIN 38 412, Teil 25 bestimmt.

Die Polyurethane 1 bis 5 gehören zum Stand der Technik, während die Polyurethane 6 und 7 neue Stoffe gemäß Erfindung sind.

Um die Verwendung als Haarbehandlungsmittel zu demonstrieren, wurden folgende Haarbehandlungsmittel hergestellt:
(a) Aerosol-Haarspray (rein ethanolisch)

| | |
|---|---|
| Polyurethan gemäß Beispiel 3 | 3% |
| 2-Amino-2-methyl-propanol | 0,26% |
| Ethanol abs. | 61,74% |
| Dimethylether | 35 % |

(b) Aerosol-Haarspray (wäßrig-alkoholisch)

| | |
|---|---|
| Polyurethan gemäß Beispiel 3 | 3,00% |
| 2-Amino-2-methyl-propanol | 0,26% |
| Wasser dest. | 10,00% |
| Ethanol abs. | 51,74% |
| Dimethylether | 35,00% |

(c) Handpumpenspray

| | |
|---|---|
| Polyurethan gemäß Beispiel 3 | 6,00% |
| 2-Anino-2-methyl-propanol | 0,52% |
| Wasser dest. | 93,48% |

(d) Haarfestiger (rein wäßrig)

| | |
|---|---|
| Polyurethan gemäß Beispiel 5 | 4,00% |
| 2-Amino-2-methyl-propanol | 0,37% |
| Wasser dest. | 95,63% |

(e) Haarfestiger (wäßrig-alkoholisch)

| | |
|---|---|
| Polyurethan gemäß Beispiel 5 | 4,00% |
| 2-Amino-2-methyl-propanol | 0,37% |
| Wasser dest. | 63,75% |
| Ethanol abs. | 31,88% |

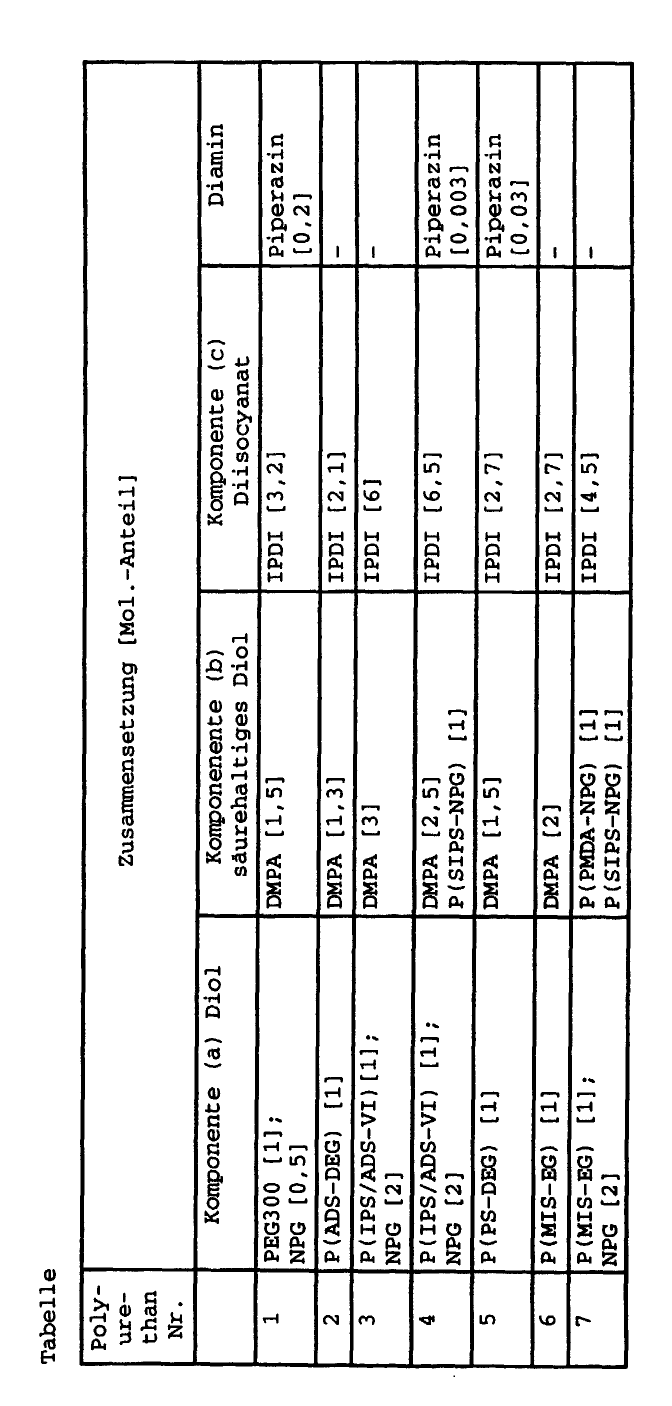

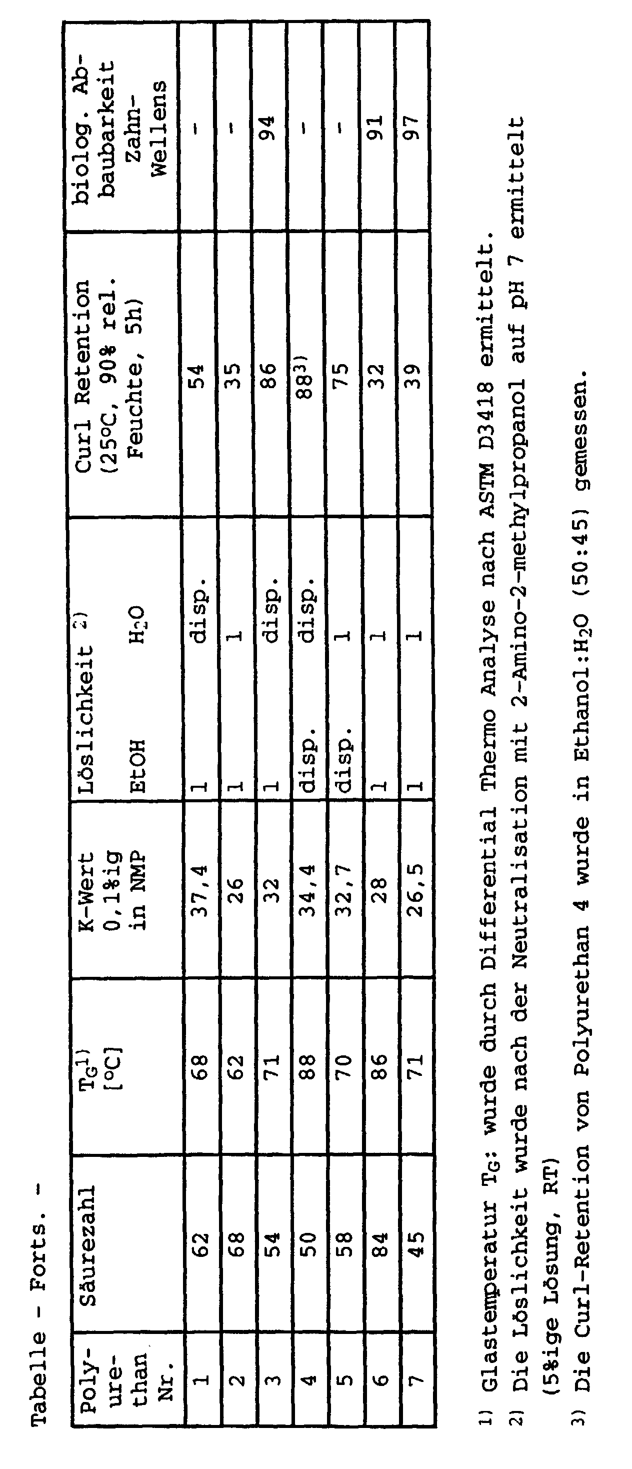

## Patentansprüche

1. Wasserlösliche oder in Wasser dispergierbare Polyurethane aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat
mit Säurezahlen von 12 bis 150 oder den Salzen dieser Polyurethane, dadurch gekennzeichnet, daß sie als Verbindungen der Gruppe (a) mindestens 5 mol-% eines Polykondensats aus Milchsäure und einem Polyol der allgemeinen Formel 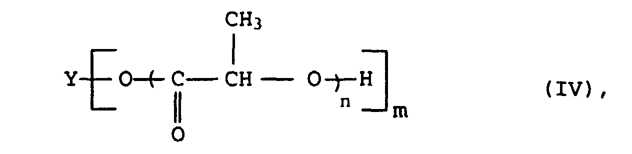 in der
y Rest eines 2- bis 4-wertigen Alkohols,
n 1 - 50 und
m 1 - 4 bedeutet,
einpolymerisiert enthalten.

2. Verfahren zur Herstellung der wasserlöslichen oder in Wasser dispergierbaren Polyurethane nach Anspruch 1, dadurch gekennzeichnet, daß man die Verbindungen der Gruppen (a) und (b) unter einer Inertgasatmosphäre in einem inerten Lösemittel bei Temperaturen von 70 bis 130°C mit den Verbindungen der Gruppe (c) umsetzt, wobei gegebenenfalls übliche Kettenverlängerer mitverwendet werden.

3. Verwendung von Polyurethanen gemäß Anspruch 1 in kosmetischen Zubereitungen.

4. Verwendung nach Anspruch 3 als Haarpflegemittel.

5. Verwendung von Polyurethanen gemäß Anspruch 1 als Tablettenüberzugsmittel oder Tablettenbinder.

6. Verwendung von Polyurethanen gemäß Anspruch 1 als Klebstoffe.

## Claims

1. A polyurethane which is soluble or dispersible in water and is composed of
a) at least one compound which contains two or more active hydrogens per molecule,
b) at least one diol containing acid groups or salt groups and
c) at least one diisocyanate
with acid numbers of from 12 to 150 or the salts of this polyurethane, which contains as compounds in group (a) at least 5 mol % of a polycondensate of lactic acid and of a polyol of the formula 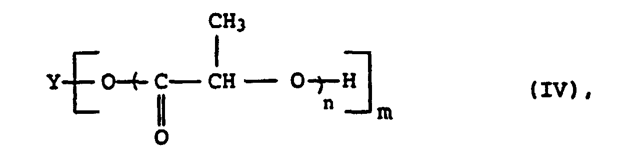 where
Y is a radical derived from a dihydric to tetrahydric alcohol,
n is 1 - 50 and
m is 1 - 4,
as copolymerized units.

2. A process for preparing the polyurethanes which are soluble or dispersible in water as claimed in claim 1, which comprises reacting the compounds in groups (a) and (b) under an inert gas atmosphere in an inert solvent at from 70 to 130°C with the compounds in group (c), also using where appropriate conventional chain extenders.

3. The use of polyurethanes as claimed in claim 1 in cosmetic compositions.

4. The use as claimed in claim 3 as hair care compositions.

5. The use of polyurethanes as claimed in claim 1 as tablet coating compositions or tablet binders.

6. The use of polyurethanes as claimed in claim 1 as adhesives.

## Revendications

1. Polyuréthannes solubles dans l'eau ou dispersables dans l'eau, constitués de
a) au moins un composé contenant deux ou plusieurs atomes d'hydrogène actif par molécule,
b) au moins un diol contenant des groupes acides ou salins et
c) au moins un diisocyanate,
à des indices d'acides de 12 à 150, ou les sels de ces polyuréthannes, caractérisés par le fait qu'ils contiennent à l'état polymérisé, en tant que composé du groupe (a) et en proportions d'au moins 5 mol %, un polycondensat de l'acide lactique et d'un polyol de formule générale 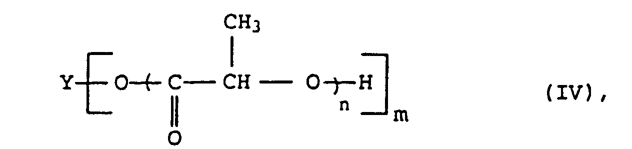 dans laquelle
Y représente le radical d'un alcool di- à tétra-valent,
n est un nombre allant de 1 à 50 et
m un nombre allant de 1 à 4.

2. Procédé de préparation des polyuréthannes solubles dans l'eau ou dispersables dans l'eau selon la revendication 1, caractérisé par le fait que l'on fait réagir les composés des groupes (a) et (b) en atmosphère de gaz inerte, dans un solvant inerte, à des températures de 70 à 130°C, avec les composés du groupe (c), en utilisant conjointement le cas échéant des agents d'allongement des chaînes de type courant.

3. Utilisation des polyuréthannes selon la revendication 1 dans des compositions cosmétiques.

4. Utilisation selon la revendication 3, sous la forme de compositions pour les soins des cheveux.

5. Utilisation des polyuréthannes selon la revendication 1 en tant que produits de revêtement pour comprimés ou liant pour comprimés.

6. Utilisation des polyuréthannes selon la revendication 1 en tant qu'adhésifs.
